# EUROPÄISCHE PATENTSCHRIFT

(11) **EP 3 199 145 B1**
(45) Veröffentlichungstag und Bekanntmachung des Hinweises auf die Patenterteilung: **17.10.2018**
(21) Anmeldenummer: 16153154.6
(22) Anmeldetag: 28.01.2016
(51) Int. Cl.: A61K 9/16, A61K 47/50, A61K 31/00, A61K 31/295, A61K 31/375, A61K 33/26

(54) **STABILISIERTE FORMULIERUNG EINES FOLSÄURE/EISEN-PRÄPARATS**
STABILIZED FORMULATION OF A FOLIC ACID/IRON PREPARATION
FORMULE STABILISEE D'UNE PREPARATION DE FER/D'ACIDES FOLIQUES

(43) Veröffentlichungstag der Anmeldung: 02.08.2017
(73) Patentinhaber: G.L. Pharma GmbH, 8502 Lannach (AT)
(72) Erfinder: BERGMANN, Kerstin, 8510 Stainz (AT); LECHNER, Doris, 8010 Graz (AT); WACHTER, Christof, 8502 Lannach (AT); WAGNER, Harald, 8055 Graz (AT); WINKLER, Heimo, 8045 Graz (AT)
(74) Vertreter: Sonn & Partner Patentanwälte

(56) Entgegenhaltungen:
- WO-A1-01/12163
- WO-A1-2005/053715
- US-A- 4 945 083
- US-A1- 2009 035 385

## Beschreibung

Das Gebiet der vorliegenden Erfindung ist das der Arzneimittelformulierung, insbesondere von Folsäure/Eisen-Präparaten zur oralen Verabreichung.

Eisen ist ein essentielles Spurenelement des Organismus. Als Coenzym der Cytochromoxidase, Katalase und Peroxidase und als Bestandteil von Hämoglobin, Myoglobin und Cytochromen ist es an zahlreichen Stoffwechselvorgängen beteiligt.

Folsäure (Vitamin B₉) muss dem Organismus in ausreichendem Maße zugeführt werden. Folsäure ist die Vorstufe des Coenzyms Tetrahydrofolsäure, welches als Carrier von C₁-Gruppen an der Biosynthese von Purinen und am Aminosäure-Stoffwechsel beteiligt ist. Damit besitzt Folsäure eine zentrale Bedeutung für den Intermediärstoffwechsel aller lebenden Zellen.

Bei dafür anfälligen Personen, z.B. bei Schwangeren, kann es zu einem Mangel an Eisen und Folsäure kommen. Dies kann unter anderem Anämie bei der Person zur Folge haben, mit Symptomen wie Müdigkeit, Depression, Blässe, Atemnot oder Herzklopfen. Im Stand der Technik sind verschiedene Folsäure/Eisen-Präparate bekannt, die gegen einen Mangel an Folsäure und Eisen eingesetzt werden können. Kombinationspräparate haben den Vorteil, dass sie die Compliance des Patienten erhöhen, weil die Einnahme gegenüber der Einnahme der jeweiligen Einzelpräparate weniger aufwändig ist.

Beispielsweise offenbart die WO 01/39601 A1 ein kaubares Nahrungsergänzungsmittel, das neben anderen Vitaminen und Mineralstoffen auch Folsäure und Eisen(II)-Fumarat umfasst (vgl. Tabelle II, S. 44, des Dokuments).

Am deutschsprachigen Arzneimittelmarkt sind Präparate verfügbar, die Folsäure und ein Eisensalz beinhalten. Beispielsweise enthält eine Tablette Ferro-Folsan® (Pharmazeutischer Unternehmer: Desma GmbH, Mainz-Kastel, Deutschland) laut Gebrauchsinformation 100 mg Eisen(II)-sulfat und 850 µg Folsäure.

Des Weiteren ist im Stand der Technik bekannt, dass Vitamine wie Folsäure (und gegebenenfalls auch andere Wirkstoffe) instabil sind und sich daher insbesondere bei längerer Lagerung eines Präparats mit Vitaminen die Dosis der Vitamine vermindern kann. Daher werden Vitamine oft im Überschuss ("Overage"), d.h. über der eigentlich empfohlenen Dosis, zugegeben.
Dies kann erhöhte Kosten für den Hersteller zur Folge haben. Des Weiteren steht zu erwarten, dass die Akzeptanz der jeweils zuständigen Arzneimittelzulassungsbehörde für solche Dosierungs-überschüsse schwinden wird.
Somit besteht eine Aufgabe der vorliegenden Erfindung darin, eine stabilisierte Arzneimittelformulierung eines Folsäure/Eisen-Präparats zur Verfügung zu stellen, in der insbesondere die Folsäure stabilisiert ist.

Daher stellt die vorliegende Erfindung eine stabilisierte Formulierung eines Folsäure/Eisen-Präparats zur Verfügung, die Folsäure, ein pharmazeutisch verträgliches Eisen(II)-Salz und zumindest einen Hilfsstoff umfasst. Diese Formulierung ist dadurch gekennzeichnet, dass sie ferner Ascorbinsäure umfasst, und dass sie zumindest zwei separat verpresste Komponenten umfasst, wobei die erste Komponente Folsäure und die zweite Komponente pharmazeutisch verträgliches Eisen(II)-Salz beinhaltet. Im Folgenden werden die genannte erste separat verpresste Komponente bzw. die genannte zweite separat verpresste Komponente auch als die "erste, Folsäure-hältige Komponente" bzw. die "zweite, Eisen(II)-hältige Komponente" bezeichnet, oder auch einfach nur als "die erste Komponente" bzw. "die zweite Komponente".

Es hat sich im Zuge der Erfindung überraschend ergeben, dass die Folsäure in der erfindungsgemäßen Formulierung stabilisiert wird, wenn die Möglichkeit einer Wechselwirkung zwischen dem Eisen(II)-Salz und der Folsäure eingeschränkt wird (vgl. Beispiel 2). Des Weiteren hat sich überraschend herausgestellt, dass die Ascorbinsäure in der erfindungsgemäßen Formulierung zur Stabilisierung der Folsäure beiträgt (vgl. ebenfalls Beispiel 2). Eine Lösung, die Folsäure zu stabilisieren, ist deshalb die erwähnte separate Verpressung in zwei Komponenten, denn eine Verpressung verringert die (für eine Wechselwirkung anfällige) Oberfläche der ersten, Folsäure-hältigen bzw. der zweiten, Eisen(II)-hältigen Komponente im Verhältnis zu deren jeweiliger, für das Erreichen der jeweiligen Dosis nötigen Masse.

Für den Fachmann versteht sich, dass jede der hierin genannten separat verpressten Komponenten, jeweils unabhängig voneinander, in mehrfacher Ausführung (z.B. als mehrere Minitabletten oder als eine Vielzahl an Pellets) in der erfindungsgemäßen Formulierung vorhanden sein kann.

Im Gegensatz zur Lehre der vorliegenden Erfindung enthalten Folsäure/Eisen-Präparate im Stand der Technik oftmals sowohl Folsäure als auch ein Eisen(II)-Salz als Granulat oder Granulatgemisch in derselben Kapsel (also mit jeweils großen, für Wechselwirkungen anfälligen Oberflächen der Folsäure- bzw. Eisen(II)-Partikel) oder sogar verpresst in derselben Komponente, wie z.B. in der WO 2006/094782 A2 offenbart. Jenes Dokument bezieht sich auf eine Zusammensetzung für die perorale Applikation mit gesteuerter Freisetzung von Wirkstoffen, insbesondere aus der Gruppe von Vitaminen, Mineralstoffen und Spurenelementen (S. 1, Zeilen 1-11 des Dokuments). Gemäß einer besonderen Ausgestaltung kann jene Zusammensetzung drei Freisetzungssysteme enthalten (S. 10, Zeilen 13-29 des Dokuments); Folsäure und Eisensalze, insbesondere Eisen(II)-Fumarat, werden als im selben Freisetzungssystem, nämlich "Freisetzungssystem B", befindlich offenbart (S. 10, Zeilen 21-25 des Dokuments). Ferner wird offenbart, dass jedes Freisetzungssystem für sich granuliert wird und die resultierenden Granulate anschließend homogen miteinander vermischt werden und zu Minitabletten verpresst werden (S. 16, Zeile 30-35). Zusammenfassend offenbart die WO 2006/094782 A2 also die gemeinsame Granulierung und Verpressung von Folsäure und Eisen(II)-Salz - im Gegensatz zur Lehre der vorliegenden Erfindung.

Eine Tablette wird durch die Verpressung eines Pulvers bzw. Granulats (bzw. Gemischen davon) hergestellt. Das Granulieren eines Pulvers (bzw. einer Pulvermischung), wie es in der Arzneimittelherstellung zur Verbesserung der Fließfähigkeit durchgeführt wird, wird nicht als Verpressung bezeichnet. Das Verpressen von Pulver zu einer Tablette, ohne dass zuvor eine Granulierung durchgeführt wurde, wird als Direktverpressung bezeichnet. Die "separate Verpressung" wird im Sinne der Erfindung gemäß einer der folgenden beiden Varianten umgesetzt:
Variante I: Eine erste Komponente, z.B. Granulat A, wird verpresst (z.B. zu einer Minitablette) und unabhängig davon (d.h. in einem anderen Arbeitsschritt oder gar in einer anderen Vorrichtung für die Verpressung) wird eine zweite Komponente, z.B. Granulat B, verpresst (z.B. zu einer Minitablette). Durch die jeweilige Verpressung entsteht eine erste separat verpresste Komponente und eine zweite separat verpresste Komponente, die dann zu einer Formulierung (also einer einzigen Verabreichungsform) kombiniert werden. Als Beispiel sei das Zusammenfügen von jeweils einer Minitablette (der ersten separat verpressten Komponente bzw. der zweiten separat verpressten Komponente) in eine Kapsel genannt.
Variante II: Eine erste Komponente, z.B. Pellets A, wird gemeinsam mit einer zweite Komponente, die von einer Beschichtung umgeben ist, z.B. mit Film überzogene Pellets B, verpresst. Hierbei ist allerdings zwingend vorausgesetzt, dass die Beschichtung auch nach der Verpressung im Wesentlichen erhalten bleibt (z.B. mindestens 80% oder mindestens 90% der Beschichtungsfläche), so dass sich im Wesentlichen keine Grenzfläche zwischen der ersten Komponente und der zweiten Komponente bildet. Durch die Verpressung entsteht eine erste separat verpresste Komponente und eine zweite separat verpresste Komponente, die bereits durch die Verpressung zu einer Formulierung (also einer einzigen Verabreichungsform) kombiniert werden. Als Beispiel sei das Verpressen von mit Film überzogenen Pellets (der ersten beschichteten Komponente und der zweiten beschichteten Komponente) zu einer Tablette genannt.

Variante I ist erfindungsgemäß gegenüber Variante II zu bevorzugen, weil eine Herstellung nach Variante I weniger fehleranfällig ist, denn beispielsweise ist keine (durch Verpressung gefährdete) Beschichtung einer Komponente nötig, um die Stabilisierung zu erreichen.

Wie erwähnt soll die Oberfläche der separat verpressten Komponenten im Verhältnis zur Masse der separat verpressten Komponenten verringert werden - wobei dieser Anforderung natürlich im Lichte anderer Anforderungen an z.B. die galenischen und pharmakologischen Eigenschaften der Formulierung nur in für den Fachmann sinnvoll erscheinenden Maßen entsprochen werden kann. Daher haben die erste separat verpresste Komponente bzw. die zweite separat verpresste Komponente der erfindungsgemäßen Formulierung, unabhängig voneinander, vorzugsweise ein Oberflächezu-Masse-Verhältnis von weniger als 4 mm²/mg, bevorzugt weniger als 3 mm²/mg, mehr bevorzugt weniger als 2,5 mm²/mg, noch mehr bevorzugt weniger als 2 mm²/mg, insbesondere weniger als 1,75 mm²/mg. Wie erwähnt, kann eine separat verpresste Komponente in mehrfacher Ausführung in der Formulierung vorhanden sein (z.B. in Form von mehreren der gleichen Minitablette). Allerdings ist es von Vorteil (im Sinne der Oberflächenverminderung), wenn jede der Ausführungen der ersten und/oder der zweiten separat verpressten Komponente der erfindungsgemäßen Formulierung eine gewisse Mindestmasse aufweist, bevorzugt mehr als 1 mg, mehr bevorzugt mehr als 5 mg, noch mehr bevorzugt mehr als 10 mg, insbesondere mehr als 20 mg oder gar mehr als 30 mg.

Vorteilhafterweise liegt in der erfindungsgemäßen Formulierung Ascorbinsäure in einer dritten separat verpressten Komponente vor, was weiter zur Stabilisierung beiträgt.

In einer weiteren bevorzugten Ausführungsform der vorliegenden Erfindung sind die erste, Folsäure-hältige Komponente und die zweite, Eisen(II)-hältige Komponente durch einen weiteren Inhaltsstoff räumlich voneinander getrennt. So kann beispielsweise eine (oder beide) der genannten Komponenten, vorzugsweise die zweite, Eisen(II)-hältige Komponente, mit dem genannten, weiteren Inhaltsstoff (z.B. einem Polymer, wie Polyvinylacetat) gegebenenfalls als Film beschichtet sein. Auch dies trägt zur Stabilisierung, insbesondere der Folsäure, bei. Vorzugsweise ist als weiterer Inhaltsstoff, insbesondere zur Beschichtung, ein Stoff vorgesehen, ausgewählt aus Hydroxypropyl-methylcellulose, Carboxymethylcellulose, Hydroxyethylcellulose, Ethylhydroxyethylcellulose, Hydroxypropylcellulose, Celluloseacetylphathalat, Hydroxypropylmethylcellulosephthalat, Celluloseacetat, Cellulosepropionat, Celluloseacetatbutyrat, Polymere der Acrylsäure und deren Salze bzw. Polyacrylate bzw. Polymethacrylate bzw. Co-Polymere davon (z.B. Eudragit® E, Eudragit® R, Eudragit® S, Eudragit® NE, Eudragit® RS, Eudragit® RL), Polymethacrylate, Polyvinylpyrrolidon, Polyvinylacetat-phthalat, natürliche Arten von Gummi wie Carrageenan, Zein und Schellack; bzw. Kombinationen davon.

Weiterhin ist insbesondere Ascorbinsäure dieser "weitere Inhaltsstoff" und trägt zur zumindest durch die räumliche Trennung der ersten Komponente von der zweiten Komponente zur erfindungsgemäßen Stabilisierung bei.

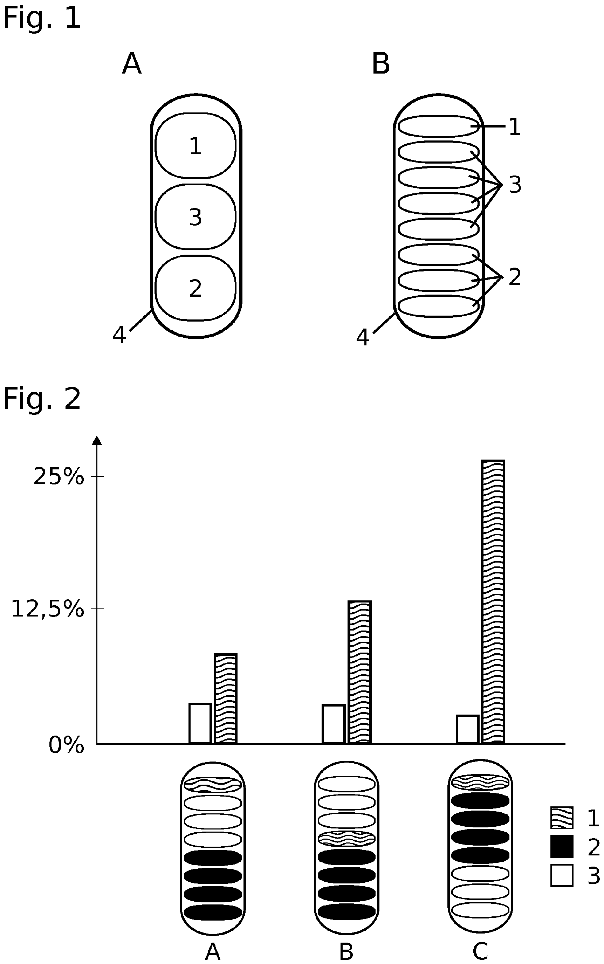

Als besonders geeignet hat sich eine Ausführungsform der erfindungsgemäßen Formulierung erwiesen, bei der Ascorbinsäure in der dritten separat verpressten Komponente vorliegt, und die erste Komponente (welche Folsäure beinhaltet) und die zweite Komponente (welche pharmazeutisch verträgliches Eisen(II)-Salz beinhaltet) durch die dritte Komponente räumlich voneinander getrennt sind (vgl. auch Fig. 1). Dies verringert allein schon durch die räumliche Trennung die Möglichkeit der Wechselwirkung zwischen Folsäure und Eisen(II)-Salz. Zusätzlich kann sich die dritte, Ascorbinsäure-hältige Komponente dadurch in räumlicher Nähe zu den anderen beiden genannten Komponenten - der ersten und der zweiten Komponente - befinden, und auf diese stabilisierend einwirken.

Es ist besonders zweckmäßig für die Stabilisierung insbesondere der Folsäure, wenn die erfindungsgemäße Formulierung als Kapsel vorliegt. Darin liegen die erste und die zweite Komponente, gegebenenfalls auch die dritte, Ascorbinsäure-hältige Komponente, bevorzugt als Minitablette(n) vor. Die besagten Komponenten liegen gegebenenfalls jeweils in mehrfacher Ausführung vor. Bevorzugt umfasst die erste, Folsäure-hältige Komponente als Hilfsstoffe ein Füllmittel wie Lactose, ein Zerfallsmittel wie mikrokristalline Cellulose, ein Fließmittel wie kolloidale Kieselsäure und/oder ein Schmiermittel wie Magnesiumstearat. Bevorzugt umfasst die zweite, Eisen(II)-hältige Komponente als Hilfsstoffe ein Füllmittel wie Lactose, ein Retardierungsmittel wie ein Copolymer von Ethylacrylat und Methylmethacrylate, ein Fließmittel wie kolloidale Kieselsäure und/oder ein Schmiermittel wie Magnesiumstearat. Die Hülle der Kapsel kann beispielsweise im Wesentlichen aus Hartgelatine bestehen. Vorzugsweise hat eine Minitablette erfindungsgemäß eine Masse zwischen 10 mg bis 100 mg, bevorzugt zwischen 20 mg bis 90 mg, mehr bevorzugt zwischen 30 mg bis 80 mg, insbesondere zwischen 40 mg und 70 mg und/oder einen Durchmesser von 0,25 mm bis 7 mm, bevorzugt von 0,5 mm - 6 mm, insbesondere von 1 mm - 5 mm.

In einer ganz besonders bevorzugten Ausführungsform der vorliegenden Erfindung ist die erfindungsgemäße Formulierung eine Kapsel, worin die erste separat verpresste, Folsäure-hältige Komponente und die zweite separat verpresste, Eisen(II)-hältige Komponente jeweils als eine oder mehrere Minitabletten vorliegen. In dieser Ausführungsform liegt Ascorbinsäure in einer dritten, separat verpressten Komponente als eine oder mehrere Minitablette(n) vor, diese dritte Komponente ist zwischen der ersten separat verpressten Komponente und der zweiten separat verpressten Komponente in der Kapsel gelegen. Die erste und die zweite separat verpresste Komponente werden somit in dieser Ausführungsform durch die dritte separat verpresste Komponente räumlich voneinander getrennt (vgl. auch Fig. 1).

In einer weiteren bevorzugten Ausführungsform der vorliegenden Erfindung liegt die erfindungsgemäße Formulierung als Tablette vor. Die erfindungsgemäße Stabilisierung wird bevorzugt dadurch erreicht, dass die erste und die zweite Komponente, gegebenenfalls auch die dritte, Ascorbinsäure-hältige Komponente, jeweils in mehrfacher Ausführung als Pellets vorliegen. Bevorzugt weist zumindest eine der genannten Komponenten, insbesondere die zweite, Eisen(II)-hältige Komponente, (gegebenenfalls in mehrfacher Ausführung) eine Beschichtung auf. Beispielsweise kann die zweite, Eisen(II)-hältige Komponente in Form von Pellets vorliegen, welche jeweils einzeln beschichtet sind (d.h. es kann auch von einer Komponente gesprochen werden, die beschichtet ist bzw. eine Beschichtung aufweist). Insbesondere liegt die erfindungsgemäße Formulierung in Form einer Tablette als Multiple Unit Pellet System (auch als MUPS® bezeichnet) vor. Multiple Unit Pellet Systeme sind beispielsweise bekannt aus der WO 2011/107921 A2, der WO 2013/092497 A1 oder der WO 2014/016396 A1.

Im Sinne der vorliegenden Erfindung kann jedes pharmazeutisch verträgliche bzw. annehmbare Eisen(II)-Salz für die erfindungsgemäße Formulierung gewählt werden. Bevorzugt ist das Eisen(II)-Salz ausgewählt aus Eisen(II)-Fumarat, -sulfat, - gluconat, -lactat, -tartrat, -succinat, -glutamat, -citrat, - pyrophosphat, -carbonat, und Kombinationen davon. Insbesondere liegt das Eisen (II) -Salz als Eisen(II)-Fumarat vor, welches sich als sehr geeignet für die Zwecke der vorliegenden Erfindung erwiesen hat.

Als Hilfsstoff umfasst die erfindungsgemäße Formulierung (bzw. eine oder alle getrennt verpressten Komponenten in der Formulierung) beispielsweise einen Stoff, ausgewählt aus der Gruppe der Polysaccharide, wie Cellulose (z.B. mikrokristalline Cellulose) Hydroxypropylmethylcellulose, Carboxymethylcellulose, Hydroxyethylcellulose, Ethylhydroxyethylcellulose, Hydroxypropyl-cellulose, Celluloseacetylphathalat, Hydroxypropylmethylcellulosephthalat, Celluloseacetat, Cellulosepropionat, Celluloseacetatbutyrat, Ethylcellulose, Guar-Mehl, Alginsäure und/oder Alginate, Pektin, Polyvinylpyrrolidon Polyvinylacetat, Polyvinylalkohol, Polyvinylchlorid, Polymere der Acrylsäure und deren Salze bzw. Polyacrylate bzw. Polymethacrylate bzw. Co-Polymere davon (z.B. Eudragit® E, Eudragit® R, Eudragit® S, Eudragit® NE, Eudragit® RS, Eudragit® RL), Vinylpyrrolidon-VinylacetatCopolymere, Nylon, kolloidale Kieselsäure (z.B. Aerosil®), Behenate, Stearate, Polyamid, Polyacrylamid, Polyethylen, Polyalkylenglykole wie Polyethylenglykol, Co-polymere von Polyalkylenglykolen, z.B. von Polyethylenglykol und Polypropylenglykol (Pluronic®, BASF), Zucker, Zuckeralkohol; und Gemischen davon. Dem Fachmann sind weitere geeignete Hilfsstoffe vertraut.

Eine weitere Aufgabe der vorliegenden Erfindung ist, eine Formulierung eines Folsäure/Eisenpräparats zur Verfügung zu stellen, die bei Einnahme möglichst geringe Nebenwirkungen verursacht bzw. eine möglichst effektive Resorption der darin beinhalteten Folsäure bzw. des Eisen(II)-Salzes im Körper des Patienten bewirkt. Bei der Einnahme eines Eisen/Folsäure-Präparats können im Verdauungstrakt hohe lokale Eisenkonzentrationen entstehen, welche mitunter die Schleimhaut im Verdauungstrakt reizen und dadurch z.B. Übelkeit verursachen können. Daher liegt in einer weiteren, besonders bevorzugten Ausführungsform der vorliegenden Erfindung die zweite, Eisen(II)-hältige Komponente in retardierter Form in der Formulierung vor.

Wie ein Wirkstoff in retardierter Form formuliert werden kann (d.h. mit verzögerter Wirkstofffreisetzung formuliert werden kann), ist im Stand der Technik bekannt, beispielsweise aus der WO 2010/028794 A1 oder der WO 2010/105824 A1. Bevorzugt wird ein Polymer in der erfindungsgemäßen Formulierung als retardierender Hilfsstoff eingesetzt, welches Polymer ausgewählt ist aus den neutralen Homo- und Co-polymeren aus (Meth)acrylsäureestern, kationischen Homo- und Co-polymeren aus (Meth)acrylsäureestern mit quartären Ammoniumgruppen, Polyvinylacetat, Celluloseacetat, Cellulosepropionat, Celluloseacetatbutyrat, Ethylcellulose, bevorzugt aus neutralen Homo- und Co-polymeren aus (Meth)acrylsäureestern, insbesondere einem Co-Polymer aus Ethylacrylat und Methylmethacrylat. Es ist vorteilhaft, wenn die Formulierung bzw. die zweite, Eisen(II)-hältige Komponente, wenn sie in retardierter Form vorliegt, 50% des Eisen(II)-Gehalts erst nach mehr als 10 Minuten freigesetzt hat, bevorzugt erst nach mehr als 30 Minuten, mehr bevorzugt erst nach mehr als 50 Minuten, noch mehr bevorzugt erst nach mehr als 70 Minuten, insbesondere erst nach mehr als 80 Minuten oder gar erst nach mehr als 90 Minuten; wobei es gegebenenfalls von Vorteil ist, wenn der Eisen(II)-Gehalt nach weniger als 36 Stunden im Wesentlichen vollständig freigesetzt worden ist, bevorzugt nach weniger als 24 Stunden, mehr bevorzugt nach weniger als 18 Stunden, noch mehr bevorzugt nach weniger als 14 Stunden, insbesondere nach weniger als 12 oder gar nach weniger als 10 Stunden. Bevorzugt wird die Freisetzung von Eisen(II) nach dem in Beispiel 3 offenbarten Verfahren gemessen, wobei natürlich unter anderem die Zeitpunkte der Probenentnahmen auf dem Fachmann zweckmäßig erscheinende Weise variiert werden können.

Die zweite, Eisen(II)-hältige Komponente der erfindungsgemäßen Formulierung weist, wenn sie in retardierter Form vorliegt, vorteilhafterweise eine nicht-quellbare Matrix auf (d.h. die Matrix schwillt in wässriger Lösung nicht auf). Die Matrix der zweiten, Eisen(II)-hältigen Komponente hat vorzugsweise eine Quellungszahl, die unter 1,5, bevorzugt unter 1,4, mehr bevorzugt unter 1,3, noch mehr bevorzugt unter 1,2, insbesondere unter 1,1 oder gar unter 1,05 liegt, z.B. bei etwa 1. Die Quellungszahl gibt das Volumen in Millilitern an, das 1 g Stoff einschließlich des gegebenenfalls anhaftenden Schleims nach Quellen in einer wässrigen Lösung nach 4 Stunden einnimmt. Die Quellungszahl wird nach Ph. Eur. 8. Ausgabe, Kapitel 2.8.4 bestimmt.

Wider Erwarten hat sich herausgestellt, dass die Formulierung besonders gut für die erfindungsgemäßen Zwecke geeignet ist, wenn Ascorbinsäure nicht retardiert in der Formulierung vorliegt, insbesondere wenn Eisen(II)-Salz retardiert vorliegt. Mit anderen Worten: Es ist besonders vorteilhaft, wenn Ascorbinsäure nicht gemeinsam mit dem Eisen(II)-Salz in der zweiten, separat verpressten Komponente vorliegt, wenn diese retardiert ist. Daher liegt einer weiteren, besonders bevorzugten Ausführungsform der vorliegenden Erfindung Ascorbinsäure bzw. die dritte separat verpresste Komponente, welche Ascorbinsäure beinhaltet, in nicht-retardierter Form in der Formulierung vor. Ferner liegt in einer weiteren, bevorzugten Ausführungsform der vorliegenden Erfindung Folsäure bzw. die erste separat verpresste Komponente, welche Folsäure beinhaltet, in nicht-retardierter Form in der Formulierung vor.

Es hat sich gezeigt, dass Eisen(II)-Salz besonders gut nach der Einnahme resorbiert wird, wenn dieser Prozess nicht durch das Vorhandensein weiterer Vitamine, Mineralstoffe und/oder Spurenelemente in der Formulierung gestört wird. Somit enthält die erfindungsgemäße Formulierung keine weiteren Stoffe, ausgewählt aus Vitaminen (z.B. Vitamine D, E, oder K), Mineralstoffen (z.B. zweiwertigen Metallsalzen wie Ca(II)-, Cu(II)-, Mn(II)- und Zn(II)-Salze) und/oder Spurenelementen (z.B. Cu, I, Mn, Se, Mo, Zn bzw. Verbindungen oder Salze davon) als pharmazeutische Wirkstoffe (für den Fachmann ist evident, dass sich die Gruppen der Mineralstoffe bzw. Spurenelemente überschneiden). Anders ausgedrückt enthält die erfindungsgemäße Formulierung in einer bevorzugten Ausführungsform als pharmazeutische Wirkstoffe im Wesentlichen nur Folsäure, ein pharmazeutisch verträgliches Eisen(II)-Salz, insbesondere Eisen(II)-Fumarat, und Ascorbinsäure.

Zur Behandlung von Eisenmangelzuständen mit FolsäureDefizit, insbesondere zur Einnahme einmal täglich, sind folgende Dosen an pharmazeutisch verträglichen Eisen(II)-Salz, Folsäure bzw. Ascorbinsäure in der erfindungsgemäßen Formulierung besonders geeignet und daher erfindungsgemäß unabhängig voneinander oder in Kombination zu bevorzugen:
Bevorzugte Dosis (d.h. Gesamtdosis) von pharmazeutisch verträglichem Eisen(II)-Salz in der erfindungsgemäßen Formulierung ist eine Dosis von pharmazeutisch verträglichem Eisen(II)-Salz, insbesondere Eisen(II)-Fumarat, entsprechend 10 mg - 130 mg, bevorzugt 30 mg - 110 mg, mehr bevorzugt 40 mg - 100 mg, noch mehr bevorzugt 50 mg - 90 mg, insbesondere 60 mg - 80 mg, oder gar 60 mg - 70 mg Fe²⁺.

Bevorzugte Dosis (d.h. Gesamtdosis) von Folsäure in der erfindungsgemäßen Formulierung ist eine Dosis von 100 µg - 1500 µg, bevorzugt 300 µg - 1300 µg, mehr bevorzugt 500 µg - 1100 µg, noch mehr bevorzugt 600 µg - 1000 µg, insbesondere 700 µg - 900 µg, oder gar 750 µg - 850 µg Folsäure.

Bevorzugte Dosis (d.h. Gesamtdosis) von Ascorbinsäure in der erfindungsgemäßen Formulierung ist eine Dosis von 10 mg - 190 mg, bevorzugt 30 mg - 170 mg, mehr bevorzugt 50 mg - 150 mg, noch mehr bevorzugt 70 mg - 130 mg, insbesondere 80 mg - 120 mg, oder gar 90 mg - 110 mg Ascorbinsäure.

Insbesondere weist die erfindungsgemäße Formulierung die jeweilige Dosis von pharmazeutisch verträglichem Eisen(II)-Salz, Folsäure bzw. Ascorbinsäure gemäß aller der drei vorigen Absätze auf.

Neben dem überraschenden stabilisierenden Effekt auf die Folsäure (vgl. Beispiel 2), verstärkt die gleichzeitige Einnahme von Ascorbinsäure auch die enterale Resorption von zweiwertigem Eisen. In herkömmlichen Präparaten wird dafür so viel Ascorbinsäure vorgesehen, dass Fe²⁺ zu Ascorbinsäure in einem Massenverhältnis von ca. 1:3 bis 1:4 im Präparat vorliegt. Es hat sich jedoch unerwartet herausgestellt, dass wesentlich weniger Ascorbinsäure für eine hinreichende Resorptionsunterstützung (z.B. das Erreichen von 80%, 85% oder gar 90% der Eisenresorption, welche beim genannten Massenverhältnis von 1:4 erreicht wird) erforderlich ist. Eine Minderung der Ascorbinsäuremenge in der Formulierung verringert das Risiko für eine Nebenwirkung bzw. die Kosten für die Herstellung. Vorteilhafterweise liegt deshalb das Massenverhältnis von Fe²⁺ zu Ascorbinsäure in der erfindungsgemäßen Formulierung bei 1:1 bis 1:2, bevorzugt bei 1:1,1 bis 1:1,9 , mehr bevorzugt bei 1:1,2 bis 1:1,8, noch mehr bevorzugt bei 1:1,3 bis 1:1,7 , insbesondere bei 1:1,4 bis 1,6 oder gar bei im Wesentlichen 1:1,5. Beispielsweise kann die Eisenresorption zwei Wochen nach der Einnahme einer ⁵⁵Fe bzw. ⁵⁹Fe-markierten Formulierung in einem Ganzkörperzähler oder an einer Blutprobe mittels eines Szintillationszählers ermittelt werden.

Die erfindungsgemäße Formulierung ist bevorzugt zur Anwendung in der Vorbeugung gegen, oder Behandlung von, Eisenmangelzuständen mit Folsäuredefizit, insbesondere vier Wochen vor der Konzeption und/oder im ersten Trimenon der Schwangerschaft.

Gerade für Schwangere (insbesondere im ersten Trimenon, weil dieser für die Neuralrohrentwicklung essentiell ist) bzw. vier Wochen vor der Konzeption (um bis zur Konzeption einen entsprechenden Spiegel an Eisen bzw. Folsäure aufzubauen) ist die erfindungsgemäße Formulierung besonders gut zur täglichen Einnahme geeignet. Ein Mangel an Eisen bzw. Folsäure in der Schwangerschaft kann verschiedene Komplikationen verursachen, die sowohl Mutter als auch das Kind betreffen. Neben Anämie umfassen diese Komplikationen vorzeitige Wehentätigkeit oder niedriges Geburtsgewicht, in schweren Fällen auch Fehlbildungen wie Neuralrohrdefekte oder Lippen-/Kiefer-/Gaumenspalten.

Ein Serum-Folatspiegel bei Erwachsenen von 7-20 nmol/L wird üblicherweise als normal erachtet, bei Werten darunter liegt ein Folsäuremangel vor. Eisenmangel wird üblicherweise indirekt durch Quantifizierung von Hämoglobin, Ferritin (wird als besonders geeignet erachtet), Erythrozyten und/oder Retikulozyten im Blut ermittelt, die Normalwerte (Referenzbereiche) für Frauen liegen gemäß einer Definition bei 12-16 g/dl Hämoglobin, 23-110 mg/dl Ferritin, 3,9-5,7 Mio./ml Erythrozyten und davon 3-18 Promille Retikulozyten.

Die erfindungsgemäße Formulierung ist bevorzugt zur peroralen Verabreichung vorgesehen, insbesondere 1x täglich.

Der Ausdruck "stabilisiert" bzw. "stabilisieren" bzw. "Stabilisierung", wie hierin gebraucht, ist im relativen Sinn zu verstehen. D.h. trotz Stabilisierung einer Substanz kann es zu einem Abbau der Substanz kommen. Die Substanz gilt auch als stabilisiert, wenn dieser Abbau gegenüber einer geeigneten Kontrolle nur verlangsamt wird.

Die vorliegende Erfindung wird durch die folgenden Beispiele und die Figuren illustriert, auf welche sie selbstverständlich nicht eingeschränkt ist.
**Figur 1****: (A)** Eine Hartgelatine-Kapsel (4) beinhaltet die erste separat verpresste, Folsäure-hältige Komponente (1) in Form einer Minitablette, die zweite separat verpresste, Eisen(II)-hältige Komponente (2) in Form einer Minitablette, und Ascorbinsäure in einer dritten separat verpressten Komponente (3) in Form einer Minitablette. Komponente (3) trennt die Komponenten (1) und (2) räumlich voneinander. **(B)** Eine Hartgelatine-Kapsel (4) beinhaltet die erste separat verpresste, Folsäure-hältige Komponente (1) in Form einer Minitablette, die zweite separat verpresste, Eisen(II)-hältige Komponente (2) in Form von vier Minitabletten, und Ascorbinsäure in einer dritten separat verpressten Komponente (3) in Form von drei Minitabletten. Komponente (3) trennt die Komponenten (1) und (2) räumlich voneinander.
**Figur 2****:** Kapseln wurden jeweils mit einer Folsäure-hältigen Minitablette (1), vier Eisen(II)-Fumarat-hältigen Minitabletten (2) und drei Ascorbinsäure-hältigen Minitabletten (3), in den Anordnungen A (Ascorbinsäure-hältige Minitabletten in der Mitte), B (Folsäure-hältige Minitablette in der Mitte) oder C (Eisen(II)-Fumarat-hältige Minitablette in der Mitte) befüllt. Nach drei Monaten wurden die Kapseln auf den Restgehalt von Folsäure bzw. Ascorbinsäure hin untersucht. Die Anordnung der Minitabletten in der Kapsel hat einen starken Einfluss auf die Stabilität der Folsäure: in Kapseln mit Anordnung A wurde in der Folsäure-hältigen Minitablette ein Folsäuregehaltsverlust (mit Wellen schraffierte Balken) von 8% festgestellt, in Kapseln mit Anordnung B ein Verlust von 13% und in Kapseln mit Anordnung C sogar ein Verlust von 26%. Im Vergleich dazu hatte die Anordnung in der Kapsel hingegen deutlich geringeren Einfluss auf den Ascorbinsäuregehalt - der Ascorbinsäuregehaltsverlust (weiße Balken) nach drei Monaten lag für alle Anordnungen zwischen 2,5% und 3,5%.

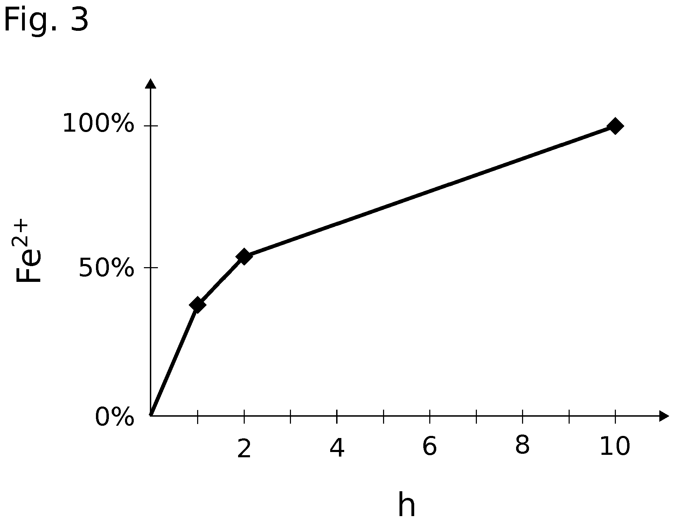

**Figur 3****:** Freisetzungsverhalten einer retardierten, gemäß Beispiel 1 hergestellten Eisen(II)-Fumarat-hältigen Minitablette in künstlichem Magensaft ohne Pepsin bei 37°C und Agitation. Nach einer Stunde waren etwa 40% des ursprünglich in der Minitablette beinhalteten Eisen (II) freigesetzt, nach zwei Stunden etwa 50% und nach 10 Stunden 100% (n=6).

### Beispiel 1 - Herstellung der Formulierung

Eine Formulierung in Form einer lichtundurchlässigen Hartgelatine-Kapsel, beinhaltend eine Folsäure-hältige Minitablette (nicht-retardiert), vier Eisen(II)-Fumarat-hältige Minitabletten (retardiert) und drei Ascorbinsäure-hältige Minitabletten (nicht-retardiert), soll hergestellt werden. Die jeweiligen Gesamtdosen in einer Kapsel sollen in etwa betragen: 800 µg Folsäure, 202, 6 mg Eisen (II) -Fumarat (entsprechend 66,6 mg Fe²⁺) (zu gleichen Teilen aufgeteilt auf 4 Minitabletten) und 100 mg Ascorbinsäure (zu gleichen Teilen aufgeteilt auf 3 Minitabletten). Hierzu wurde wie folgt verfahren:
Herstellung von 5000 Stück Folsäure-Minitabletten: 4,76g Folsäure (Gehalt 91,5%), 120,02 g Lactose, 120,23 g mikrokristalline Cellulose, 2,49 g kolloidale Kieselsäure und 2,5 g Magnesiumstearat wurden gemischt. Die so erhaltene Pulvermischung wurde zu 5000 Minitabletten zu je 50 mg direktverpresst (Stempel: 5 mm rund, Tablettendicke ca. 2 mm; Exzenterpresse Korsch EK 0, Korsch AG, Berlin).

Herstellung von 5000 Stück Eisen(II)-Fumarat-Minitabletten: 272,5 g Eisen(II)-Fumarat (Gehalt 93%) und 17,1 g Lactose-Monohydrat wurden gemischt und unter Zugabe von 77,65 g Polyacrylat Dispersion 30% (Trockenmasse: 23,3 g) granuliert. Das Granulat wurde getrocknet und gesiebt. Hernach wurden 3 g kolloidale Kieselsäure und 3 g Magnesiumstearat hinzugemischt. Das so erhaltene Granulat wurde zu 5000 Minitabletten zu je 63,8 mg direktverpresst (Stempel: 5mm rund, Tablettendicke ca. 2mm; Exzenterpresse Korsch EK 0, Korsch AG, Berlin).

Herstellung von 5000 Stück Ascorbinsäure-Minitabletten: 167g Ascorbinsäure (Gehalt 99,7%), 19,56 g Lactose, 58,67 g mikrokristalline Cellulose, 2,53 g kolloidale Kieselsäure und 2,52 g Magnesiumstearat wurden gemischt. Die so erhaltene Pulvermischung wurde zu 5000 Minitabletten zu je 50 mg direktverpresst (Stempel: 5 mm rund, Tablettendicke ca. 2 mm; Exzenterpresse Korsch EK 0, Korsch AG, Berlin).

Die Minitabletten wurden im Verhältnis 1:4:3 (Folsäure-Minitablette : Eisen(II)-Fumarat-Minitabletten : Ascorbinsäure-Minitabletten) pro Kapsel mit einer Kapselfüllmaschine in Hartgelatinekapseln verfüllt. Die Minitabletten wurden mit den Ascorbinsäure-Minitabletten in der Mitte (vgl. Fig. 2, Anordnung A) angeordnet, da diese Anordnung sehr geeignet war, um eine stabilisierte Formulierung zu erhalten.

### Beispiel 2 - Stabilitätsuntersuchungen

Kapseln wurden jeweils mit einer Folsäure-hältigen Minitablette (1), vier Eisen(II)-Fumarat-hältigen Minitabletten (2) und drei Ascorbinsäure-hältigen Minitabletten (3), in den Anordnungen A (Ascorbinsäure-hältige Minitabletten in der Mitte), B (Folsäure-hältige Minitablette in der Mitte) oder C (Eisen(II)-Fumarat-hältige Minitablette in der Mitte) befüllt (vgl. Fig. 2). Die jeweiligen Minitabletten wurden gemäß Beispiel 1 hergestellt.

Die Kapseln wurden für drei Monate unter den folgenden Umweltbedingungen in einem offen Kunststoffbehälter gelagert: Temperatur 40°C, bei 75% relativer Luftfeuchtigkeit. Danach wurden die Kapseln geöffnet und die darin befindlichen Folsäure-hältigen bzw. Ascorbinsäure-hältigen Minitabletten auf den Folsäurerestgehalt bzw. auf den Ascorbinsäurerestgehalt hin untersucht:

### Folsäurerestgehalt (bzw. Folsäuregehaltsverlust)

### Lösungen zum Herstellen:

28,6 g/L Natriumcarbonat Lösung: 28,6 g Natriumcarbonat wurden in 1000 ml H₂O dest. gelöst.
Puffer: 11,16 g Kaliumdihydrogenphosphat und 5,50 g Dikaliumhydrogenphosphat wurden in 1000 ml H₂O dest. gelöst. Es wurden 4 L Puffer hergestellt.

| | |
|---|---|
| Laufmittel (LM): | 3520 ml Puffer |
| | 480 ml Methanol |

### Probelösung:

10 Folsäure-hältige Minitabletten wurden einzeln gewogen und in einen 20 ml Maßkolben überführt. 1 ml einer 28,6 g/L Na₂CO₃-Lösung wurden dazupipettiert und mit Laufmittel bis zur Marke aufgefüllt. Danach wurde die Probe für 15 min ins Ultraschallbad gestellt. Dann wurde die Probe durch einen 0,45 µm PA Spritzenfilter in HPLC-Vials filtriert. Es wurden zwei Probelösungen hergestellt.

### Standardlösung:

10,0 mg Folsäure Referenzsubstanz, genau gewogen, wurden in einen 25 ml Maßkolben überführt und in 1,25 ml einer 28,6 g/L Na₂CO₃-Lösung gelöst. Mit Laufmittel wurde bis zur Marke aufgefüllt und für 15 min ins Ultraschallbad gestellt. Danach wurde durch einen 0,45 µm PA Spritzenfilter in HPLC-Vials filtriert. Es wurden zwei Standardlösungen hergestellt.

### HPLC-Bedingungen:

| | | |
|---|---|---|
| Säule: | PG 101 Agilent® LiChrospher® 100RP-8,5 µm; 250x4 mm | |
| LM: | LM A: | 88 VT Puffer 90% LM B: Methanol 10% |
| | | 12 VT MeOH |
| Fluss: | | 0,6 ml/min |
| Detektion: | | 280 nm |
| Injektionsvolumen: | | 5 µl |
| Auto sampler: | | 5°C |
| Run Time: | 20 min | |

### Ascorbinsäurerestgehalt (bzw. Ascorbinsäuregehaltsverlust)

### Lösungen zum Herstellen:

Phosphatpuffer: 13,6 g Kaliumdihydrogenphosphat werden in 2000 ml H₂O dest. gelöst.

| | |
|---|---|
| Laufmittel: | 1000 ml Phosphatpuffer |
| | 3000 ml Acetonitril |

### Probelösung:

10 Ascorbinsäure-hältige Minitabletten in einen 100 ml Maßkolben genau gewogen, mit Laufmittel bis zur Marke auffüllen und 15 min ins Ultraschallbad geben, durch einen 0,45 µm PA Spritzenfilter in HPLC-Vials filtrieren. Es wurden 2 Probelösungen hergestellt.

### Standardlösung:

166,5 mg Ascorbinsäure (W=100%) in einen 50 ml Maßkolben eingewogen, mit Laufmittel bis zur Marke aufgefüllt und 15 min ins Ultraschallbad gestellt. Durch einen 0,45 µm PA Spritzenfilter in HPLC-Vials filtrieren. Es wurden zwei Standardlösungen hergestellt.

### HPLC-Bedingungen:

| | |
|---|---|
| Säule: | PG 100 Macherey-Nagel Nucledur® 100-5 NH2-RP |
| Fluss: | 1,0 ml/min |
| Detektion: | 210 nm |
| Injektionsvolumen: | 20 µl |
| Auto sampler: | 5°C |
| Run Time: | 35 min |

### Resultate

Die Anordnung der Minitabletten in der Kapsel hatte einen starken Einfluss auf die Stabilität der Folsäure: In Kapseln mit den Ascorbinsäure-hältigen Minitabletten in der Mitte ("Anordnung A") wurde in der Folsäure-hältigen Minitablette ein Folsäuregehaltsverlust von 8% festgestellt, in Kapseln mit der Folsäure-hältigen Minitablette in der Mitte ("Anordnung B") ein Verlust von 13% und in Kapseln mit den Eisen(II)-Fumarat-hältigen Minitabletten ("Anordnung C") sogar ein Verlust von 26%. Im Vergleich dazu hatte die Anordnung in der Kapsel hingegen deutlich geringeren Einfluss auf den Ascorbinsäuregehalt - der Ascorbinsäuregehaltsverlust nach drei Monaten lag für alle Anordnungen zwischen 2,5% und 3,5%. Die Ergebnisse der Stabilitätsuntersuchungen sind auch in Fig. 2 dargestellt.

Daraus sind die folgenden, im Lichte des Standes der Technik überraschenden Rückschlüsse zu ziehen: Zwischen Folsäure und Eisen(II)-Salzen kommt es bei räumlicher Nähe bzw. Grenzflächenbildung zu Wechselwirkungen, welche die Folsäure destabilisieren (siehe Fig. 2, Anordnungen B und C). Gleichzeitige Nähe bzw. Grenzflächenbildung der Folsäure mit Ascorbinsäure mildert jedoch diese destabilisierende Wechselwirkung ab (siehe Fig. 2, Anordnung B), die Ascorbinsäure hat also einen zusätzlichen stabilisierenden Effekt auf die Folsäure. In dieser Vergleichsserie am stabilsten ist eine Anordnung der Minitabletten in der Kapsel gemäß Fig. 2, Anordnung A.

### Beispiel 3 - Freisetzungsverhalten der retardierten Eisen(II)-Fumarat-hältigen Minitablette

Eine gemäß Beispiel 1 hergestellte Eisen(II)-Fumarat-hältige Minitablette wurde in sechsfachem Ansatz in einem Van-Kel® VK-7000 Lösungssystem (Varian, Inc.) auf ihr Freisetzungsverhalten in untersucht. Die Bedingungen für jeden Ansatz waren: 900 ml künstlicher Magensaft ohne Pepsin nach Ph. Eur. als Medium zur Lösung der Minitablette, Rühren mit Paddel bei 100 Umdrehungen pro Minute und einer Temperatur von 37°C. Nach ein, zwei und zehn Stunden wurden Proben aus dem Medium gezogen und auf den Eisen(II)-Gehalt hin untersucht. Der Messung der Konzentration von Eisen(II) in den Proben erfolgte mittels kolorimetrischem 2,2'-Bispyridin-Nachweis.

Nach einer Stunde waren etwa 40% des ursprünglich in der Minitablette beinhalteten Eisen(II) freigesetzt, nach zwei Stunden etwa 50% und nach 10 Stunden 100%.

## Patentansprüche

1. Stabilisierte Formulierung eines Folsäure/Eisen-Präparats, umfassend Folsäure, ein pharmazeutisch verträgliches Eisen(II)-Salz und zumindest einen Hilfsstoff,
**dadurch gekennzeichnet, dass** die Formulierung ferner Ascorbinsäure umfasst, und
dass die Formulierung zumindest zwei separat verpresste Komponenten umfasst, wobei die erste Komponente Folsäure und die zweite Komponente pharmazeutisch verträgliches Eisen(II)-Salz beinhaltet.

2. Formulierung gemäß Anspruch 1, **dadurch gekennzeichnet, dass** die erste Komponente und die zweite Komponente durch einen weiteren Inhaltsstoff räumlich voneinander getrennt sind.

3. Formulierung gemäß Anspruch 2, **dadurch gekennzeichnet, dass** Ascorbinsäure in einer dritten separat verpressten Komponente vorliegt, und die erste Komponente und die zweite Komponente durch die dritte Komponente räumlich voneinander getrennt sind.

4. Formulierung gemäß einem der vorhergehenden Ansprüche, **dadurch gekennzeichnet, dass** sie als Kapsel vorliegt.

5. Formulierung gemäß Anspruch 4, **dadurch gekennzeichnet, dass** besagte Komponenten als Minitabletten vorliegen.

6. Formulierung gemäß einem der Ansprüche 1 bis 3, **dadurch gekennzeichnet, dass** sie als Tablette vorliegt, und wobei zumindest eine der ersten oder zweiten Komponente beschichtet ist, insbesondere wobei die Formulierung als Multiple Unit Pellet System vorliegt.

7. Formulierung gemäß einem der vorhergehenden Ansprüche, **dadurch gekennzeichnet, dass** das Eisen(II)-Salz als Eisen(II)-Fumarat vorliegt.

8. Formulierung gemäß einem der vorhergehenden Ansprüche, **dadurch gekennzeichnet, dass** die zweite, Eisen(II)-hältige Komponente in retardierter Form vorliegt.

9. Formulierung gemäß Anspruch 8, **dadurch gekennzeichnet, dass** Ascorbinsäure in nicht-retardierter Form vorliegt und/oder wobei die erste, Folsäure-hältige Komponente in nicht-retardierter Form vorliegt.

10. Formulierung gemäß einem der vorhergehenden Ansprüche, **dadurch gekennzeichnet, dass** sie keine weiteren Vitamine, Mineralstoffe und/oder Spurenelemente als pharmazeutische Wirkstoffe enthält.

11. Formulierung gemäß einem der vorhergehenden Ansprüche, **dadurch gekennzeichnet, dass** die darin vorhandene Dosis des pharmazeutisch verträglichen Eisen(II)-Salzes 10 mg - 130 mg, bevorzugt 30 mg - 110 mg, mehr bevorzugt 40 mg - 100 mg, noch mehr bevorzugt 50 mg - 90 mg, insbesondere 60 mg - 80 mg, oder gar 60 mg - 70 mg Fe²⁺ entspricht.

12. Formulierung gemäß einem der vorhergehenden Ansprüche, **dadurch gekennzeichnet, dass** die darin vorhandene Dosis der Folsäure 100 µg - 1500 µg, bevorzugt 300 µg - 1300 µg, mehr bevorzugt 500 µg - 1100 µg, noch mehr bevorzugt 600 µg - 1000 µg, insbesondere 700 µg - 900 µg, oder gar 750 µg - 850 µg beträgt.

13. Formulierung gemäß einem der vorhergehenden Ansprüche, **dadurch gekennzeichnet, dass** die darin vorhandene Dosis der Ascorbinsäure 10 mg - 190 mg, bevorzugt 30 mg - 170 mg, mehr bevorzugt 50 mg - 150 mg, noch mehr bevorzugt 70 mg - 130 mg, insbesondere 80 mg - 120 mg, oder gar 90 mg - 110 mg beträgt.

14. Formulierung gemäß einem der vorhergehenden Ansprüche, **dadurch gekennzeichnet, dass** das Massenverhältnis von Fe²⁺ zu Ascorbinsäure in der Formulierung bei 1:1 bis 1:2 liegt, bevorzugt bei 1:1,1 bis 1:1,9 , mehr bevorzugt bei 1:1,2 bis 1:1,8, noch mehr bevorzugt bei 1:1,3 bis 1:1,7 , insbesondere bei 1:1,4 bis 1,6 oder gar bei im Wesentlichen 1:1,5.

15. Formulierung gemäß einem der vorhergehenden Ansprüche zur Anwendung in der Vorbeugung gegen oder Behandlung von Eisenmangelzuständen mit Folsäuredefizit, insbesondere vier Wochen vor der Konzeption und/oder im ersten Trimenon der Schwangerschaft.

## Claims

1. Stabilised formulation of a folic acid/iron preparation, comprising folic acid, a pharmaceutically acceptable iron (II) salt and at least one additive, **characterised in that** the formulation further comprises ascorbic acid and **in that** the formulation comprises at least two separately compressed components, the first component containing folic acid and the second component containing pharmaceutically acceptably iron (II) salt.

2. Formulation according to claim 1, **characterised in that** the first component and the second component are spatially separated from each other by an additional ingredient.

3. Formulation according to claim 2, **characterised in that** ascorbic acid is present in a third, separately compressed component, and the first component and the second component are spatially separated from each other by the third component.

4. Formulation according to any of the preceding claims, **characterised in that** it is in the form of a capsule.

5. Formulation according to claim 4, **characterised in that** said components are in the form of minitablets.

6. Formulation according to any of claims 1 to 3, **characterised in that** it is in the form of a tablet, and at least one of the first or second component being coated, in particular the formulation being in the form of a Multiple Unit Pellet System.

7. Formulation according to any of the preceding claims, **characterised in that** the iron (II) salt is in the form of iron (II) fumarate.

8. Formulation according to any of the preceding claims, **characterised in that** the second, iron (II)-containing component is present in slow-release form.

9. Formulation according to claim 8, **characterised in that** ascorbic acid is present in immediate-release form, and/or the first, folic acid-containing component being present in immediate-release form.

10. Formulation according to any of the preceding claims, **characterised in that** it does not contain any additional vitamins, mineral substances and/or trace elements as pharmaceutical active ingredients.

11. Formulation according to any of the preceding claims, **characterised in that** the dose of the pharmaceutically acceptable iron (II) salt present therein corresponds to 10 mg-130 mg, preferably 30 mg-110 mg, more preferably 40 mg-100 mg, even more preferably 50 mg-90 mg, in particular 60 mg-80 mg, or even 60 mg-70 mg Fe²⁺.

12. Formulation according to any of the preceding claims, **characterised in that** the dose of the folic acid present therein is 100 µg-1500 µg, preferably 300 µg-1300 µg, more preferably 500 µg-1100 µg, even more preferably 600 µg-1000 µg, in particular 700 µg-900 µg, or even 750 µg-850 µg.

13. Formulation according to any of the preceding claims, **characterised in that** the dose of the ascorbic acid present therein is 10 mg-190 mg, preferably 30 mg-170 mg, more preferably 50 mg-150 mg, even more preferably 70 mg-130 mg, in particular 80 mg-120 mg, or even 90 mg-110 mg.

14. Formulation according to any of the preceding claims, **characterised in that** the mass ratio of Fe²⁺ to ascorbic acid in the formulation is 1:1 to 1:2, preferably 1:1.1 to 1:1.9, more preferably 1:1.2 to 1:1.8, even more preferably 1:1.3 to 1:1.7, in particular 1:1.4 to 1.6 or even substantially 1:1.5.

15. Formulation according to any of the preceding claims for use in the prevention or treatment of iron deficiency conditions and folic acid deficiency, in particular four weeks before conception and/or in the first trimester of pregnancy.

## Revendications

1. Formulation stabilisée d'une préparation de fer/acide folique comprenant de l'acide folique, un sel de fer (II) pharmaceutiquement acceptable et au moins un excipient, **caractérisée en ce que** la formulation comprend en outre de l'acide ascorbique, et **en ce que** la formulation comprend au moins deux composants comprimés séparément, le premier composant contenant l'acide folique et le deuxième composant contenant le sel de fer (II) pharmaceutiquement acceptable.

2. Formulation selon la revendication 1, **caractérisée en ce que** le premier composant et le deuxième composant sont séparés spatialement l'un de l'autre par un autre ingrédient.

3. Formulation selon la revendication 2, **caractérisée en ce que** l'acide ascorbique se présente dans un troisième composant comprimé séparément, et le premier composant et le deuxième composant sont séparés spatialement l'un de l'autre par le troisième composant.

4. Formulation selon l'une des revendications précédentes, **caractérisée en ce qu'**elle se présente sous la forme d'une capsule.

5. Formulation selon la revendication 4, **caractérisée en ce que** lesdits composants se présentent sous forme de mini-comprimés.

6. Formulation selon l'une des revendications 1 à 3, **caractérisée en ce qu'**elle se présente sous la forme d'un comprimé, et au moins un des premier ou deuxième composant étant enrobé, la formulation se présentant notamment sous forme de système granulé unitaire multiple.

7. Formulation selon l'une des revendications précédentes, **caractérisée en ce que** le sel de fer (II) se présente sous forme de fumarate de fer (II).

8. Formulation selon l'une des revendications précédentes, **caractérisée en ce que** le deuxième composant contenant du fer (II) se présente sous une forme retardée.

9. Formulation selon la revendication 8, **caractérisée en ce que** l'acide ascorbique se présente sous une forme non retardée et/ou le premier composant contenant l'acide folique se présentant sous une forme non retardée.

10. Formulation selon l'une des revendications précédentes, **caractérisée en ce qu'**elle ne contient aucune autre vitamine, aucun autre minéral et/ou élément trace comme substances actives pharmaceutiques.

11. Formulation selon l'une des revendications précédentes, **caractérisée en ce que** la dose de sel de fer (II) pharmaceutiquement acceptable qu'elle contient correspond à 10 mg - 130 mg, de préférence 30 mg - 110 mg, plus préférentiellement 40 mg - 100 mg, encore plus préférentiellement 50 mg - 90 mg, notamment 60 mg - 80 mg, voire même 60 mg - 70 mg de Fe²⁺.

12. Formulation selon l'une des revendications précédentes, **caractérisée en ce que** la dose d'acide folique qu'elle contient atteint 100 µg - 1 500 µg, de préférence 300 µg - 1 300 µg, plus préférentiellement 500 µg - 1 100 µg, encore plus préférentiellement 600 µg - 1 000 µg, notamment 700 µg - 900 µg, voire même 750 µg - 850 µg.

13. Formulation selon l'une des revendications précédentes, **caractérisée en ce que** la dose d'acide ascorbique qu'elle contient atteint 10 mg - 190 mg, de préférence 30 mg - 170 mg, plus préférentiellement 50 mg - 150 mg, encore plus préférentiellement 70 mg - 130 mg, notamment 80 mg - 120 mg, voire même 90 mg - 110 mg.

14. Formulation selon l'une des revendications précédentes, **caractérisée en ce que** le rapport massique du Fe²⁺ sur l'acide ascorbique dans la formulation est près de 1:1 à 1:2, de préférence près de 1:1,1 à 1:1,9, plus préférentiellement près de 1:1,2 à 1:1,8, encore plus préférentiellement près de 1:1,3 à 1:1,7, notamment près de 1:1,4 à 1,6 voire même sensiblement près de 1:1,5.

15. Formulation selon l'une des revendications précédentes pour l'utilisation dans la prévention ou le traitement des états de carence en fer avec un déficit en acide folique, notamment quatre semaines avant la conception et/ou dans le premier trimestre de la grossesse.
